# EUROPEAN PATENT APPLICATION

(11) **EP 2 014 179 A1**
(43) Date of publication of application: **14.01.2009**
(21) Application number: 07013751.8
(22) Date of filing: 13.07.2007
(51) Int. Cl.: A23L 1/30, A61K 31/201

(54) **A new use of conjugated linoleic acid**

(71) Applicant: Cognis IP Management GmbH, 40589 Düsseldorf (DE)
(72) Inventor: Bell, Doris, 40627 Düsseldorf (DE); Ferrari, Paolo, 20146 Milano (IT)

(57) **Abstract**

The present invention relates to use of conjugated linoleic acid (CLA) to support the recovery of a defective intestinal flora of a mammal and to the use of CLA to keep up or support a balanced intestinal flora in a mammal.

## Description

The present invention relates to use of conjugated linoleic acid (CLA) to support the recovery of a defective intestinal flora of a mammal and to the use of CLA to keep up or support a balanced intestinal flora in a mammal. This use may be a therapeutic use or a non-therapeutic use.

The following paragraphs describe the role of intestinal microbiota in relation to food compounds and additives.

It is generally accepted that a well balanced intestinal microbiota is of significant importance for the health of the host (e.g. increased colonization resistance, stimulation of immune system, support in digestion and synthesis of nutrients, production of butyrate for the enterocytes). The indigenous gut microbiota should contain high numbers of beneficial bacteria such as lactobacilli and bifidobacteria and low numbers of potential harmful micro organisms such as clostridia and coli-like bacteria. The composition of the microbiota can be influenced by external factors, including food compounds or additives. For example, it is possible to increase the numbers of lactobacilli and bifidobacteria by certain 'prebiotics' or by feeding 'probiotics'.

Infants on breast feeding contain relatively high numbers of lactobacilli and bifidobacteria. However, infants on formula milk harbour relatively less of these beneficial micro organisms and contain more of the putrefactive and potential pathogenic bacteria, such as clostridia and enterobacteriaceae. It is commonly accepted that lactic acid bacteria such as lactobacilli and bifidobacteria are capable of reducing the numbers of harmful micro organisms. Therefore, these species are frequently tested for their potential probiotic effects. Non-digestible dietary fibers, such as fructo-oligosaccharides (FOS), have a well established prebiotic effect by acting as a specific substrate for beneficial micro organisms. A recent development is the combined use of probiotics and prebiotics, so called 'synbiotics'. The concept behind this is that i) the probiotic strains bring their own 'lunchbox' and therefore survive the transit through the gastrointestinal tract better; or ii) that both the probiotic effect and the separate effect of the prebiotics contribute to a healthy microbiota in the large intestine.

The following paragraphs describe the relation of intestinal microbiota and bioconversion.

Food compounds that are not digested in or taken up by the small intestine, reach the large intestine unaltered and can be fermented by the colonic microbiota. In the case of carbohydrates this bioconversion leads to the production of a.o. short-chain fatty acids (SCFA; mainly acetate, propionate and butyrate). These SCFA are beneficial to the host. To name just a few beneficial properties, SCFA provide energy for the host (either to the epithelial cells in the case of butyrate; or systemically in the body for propionate and acetate), they reduce the pH in the colon and thereby inhibit pathogen growth, or SCFA increase calcium uptake in the body by increasing the solubility of calcium in the colon.

Another important function of a well balanced intestinal microbiota is the breakdown of potential harmful (food) compounds. On the other hand the microbiota can produce potential harmful compounds, for example by shifting from carbohydrate fermentation to protein fermentation resulting in the formation of isoSCFA (or branched-chain fatty acids; BCFA), ammonia, indol, skatol, phenol and p-cresol, or by activating pro-carcinogens by the activity of nitro- and azoreductase. This bioconversion can change the chemical composition as well as the activity of the compound. In this way the microbiota can activate or inactivate bio-active compounds, including drugs.

Food compounds and additives which are not digested in the small intestine can influence the level of activity as well as the 'direction' of the fermentative activity of the microbiota. This can have positive or negative effects on the (short or long term) health of the host.

WO 2006/005464 discloses dietary supplements comprising prebiotics and physiologically active fatty acids, their salts or esters and their use as substituents for probiotics and for making medicaments to improve the health status of the body. The physiologically active fatty acids can be conjugated linoleic acid.

It is desirable that mammals, especially humans have a naturally balanced intestinal bacterial flora. This intestinal flora may be deteriorated for several reasons. Its viability may be reduced by antibiotics. There is a need to support the recovery of a defective intestinal flora of mammals and there is a need to keep up or to support a balanced intestinal flora in mammals. To meet this need is the problem underlying the present invention.

This problem is solved by the use of CLA to support the recovery of a defective intestinal flora of a mammal and by the use of CLA to keep up or support a balanced intestinal flora in a mammal, respectively.

CLA is an abbreviation for conjugated linoleic acid. Conjugated linoleic acid is an octadecadienoic acid, in which the two double bonds are not separated by one or more saturated carbon atoms, but in which the two double bonds are located between carbon atoms no. n and n+1 and between carbon atoms no. n+2 and n+3, wherein n is an integer that is at least two and at most 15. In one embodiment of the present invention n is 9 or 10, i. e. the conjugated linoleic acid is a 9,11-octadecadienoic acid or a 10,12-octadecadienoic acid. At each of the two double bonds cis-trans isomerism (E/Z isomerism) is possible. The isomers possible are identified by c for cis and t for trans, i. e. c9,t11-CLA is cis-9-trans-11-octadecadienoic acid. In one embodiment of the present invention CLA is present as c9,t11-CLA or as t10,c12-CLA.

According to the present invention CLA may be a free fatty acid, a salt thereof or a compound thereof. In one embodiment of the present invention CLA is present as an ester, especially as an ester of glycerol. In this case the glycerol molecules may be esterified with one, two or three molecules of CLA. The remaining OH-groups of the glycerol molecules may be present as free OH-groups or may be esterified with fatty acids other than CLA.

In one embodiment of the present invention CLA is present as an ester of glycerol, wherein all three OH-groups of the glycerol are esterified with CLA. This is a so called triglyceride of CLA.

The following are embodiments of the present invention:
1. Use of CLA to support the recovery of a defective intestinal flora of a mammal.
2. Use of CLA to keep up or support a balanced intestinal flora in a mammal.
3. The use according to any of embodiments 1 or 2 wherein the mammal is a human.
4. The use according to any of embodiments 1 to 3 wherein 1 to 10 g of CLA (calculated as free acid) are administered per individual per day.
5. The use according to any of embodiments 1 to 4 wherein the intestinal flora has been or is influenced negatively by at least one antibiotic.
6. A composition comprising CLA for use as a medicament for treating or for preventing a defective intestinal flora of a mammal.
7. The composition according to embodiment 6 wherein the mammal is a human.
8. The composition according to any of embodiments 6 or 7 wherein the use comprises administering 1 to 10 g of CLA (calculated as free acid) per individual per day.
9. The composition according to any of embodiments 6 to 8 wherein the intestinal flora has been or is influenced negatively by at least one antibiotic.

CLA helps recover the naturally balanced intestinal bacterial flora when for some reason (e.g antibiotics) is reduced the viability of the health bacterial flora is reduced. CLA is not causing any additional fermentation as soluble fibers or oligosaccharides do.

It is advantageous to use CLA at a level of 1 to 10 g per individual per day. A daily dose of 4.5 g per individual is preferred.

Regular intake of CLA helps to keep a balanced intestinal flora. It helps to reduce the volume of the belly (i. e. to keep a flat belly).

According to the present invention CLA can be used in association with LB (lactic acid bacteria such as Lactobacillus acidophilus and Bifidobacterium bifidum) or without LB. CLA may be used in powder form or in oil form.

### Examples

The influence on the microbiota and the subsequent health effects of functional food components should be investigated in order to proof possible health claims of functional foods. Although the ultimate model is the human being, this 'model' has various drawbacks, such as ethical constraints, high costs, and limitations in sampling from the small and large bowel (the site where it really happens). A dynamic laboratory model, which simulates the successive kinetic conditions in the gastrointestinal tract, including an active microbiota, was used as a good alternative.

### Dynamic in vitro models of the large intestine (TIM 2)

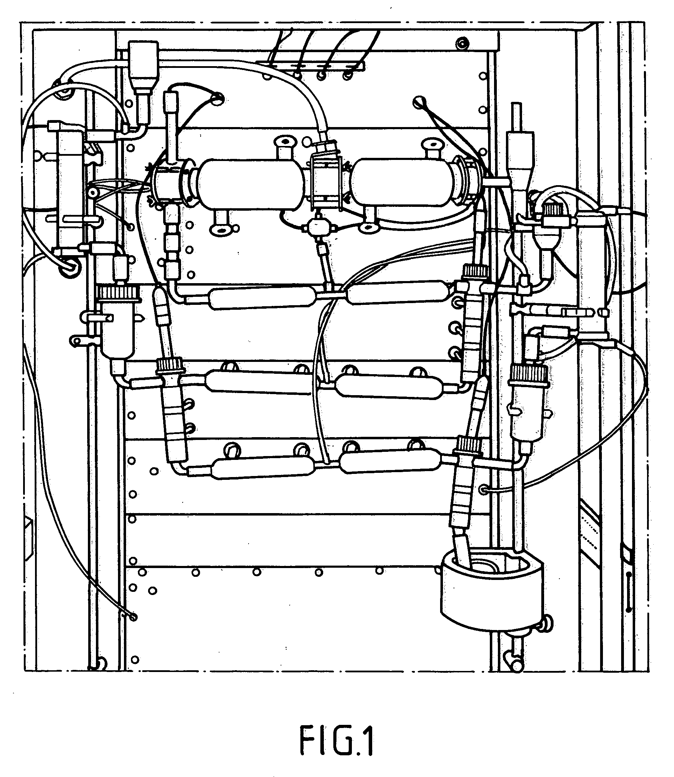

A dynamic in vitro gastrointestinal models (TIM) was developed, in which the successive conditions in the lumen of the gastrointestinal tract can be simulated in an accurate and reproducible manner (Minekus, M., Marteau, P., Havenaar, R. and Huis in 't Veld, J.H.J., A multicompartmental dynamic computer-controlled model simulating the stomach and small intestine, Alternatives to Lab. Animals 23,197-209,1995**), (**Minekus, M., Development and validation of a dynamic model of the gastrointestinal tract. PhD Thesis, University of Utrecht, Utrecht, Netherlands, 1998**), (**Havenaar, R. and Minekus, M., Simulated assimilation, Dairy Industries International 61: 17-23, 1996). This offers the opportunity to compare different ingested products under identical and standardized conditions. The model comprising the gastric and small intestinal compartments (TIM-1) is depicted in Fig. 1.

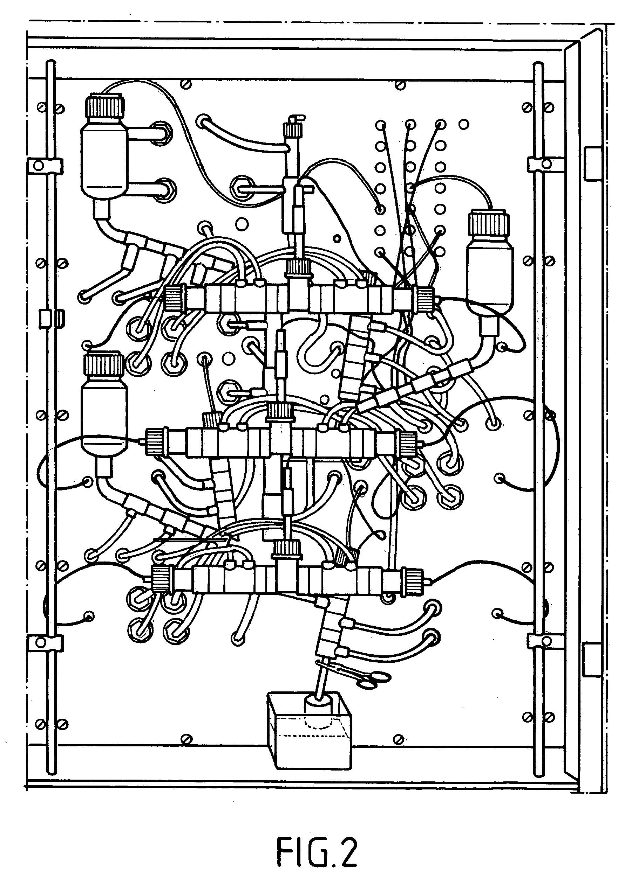

In the model comprising the large intestinal (colon) compartments (TIM 2; depicted in Fig. 2) the following standardized conditions can be simulated: body temperature, pH in the lumen, composition and rate of secretion, delivery of a pre-digested substrate from the 'ileum' mixing (peristalsis) and transport of the intestinal contents, absorption of water, complex, high density, metabolic active, anaerobic microbial flora of human (or animal) origin, and absorption of metabolic products via a semi-permeable membrane inside the colon model (Minekus, M., Smeets-Peeters, M., Bernalier, A., Marol-Bonnin, S., Havenaar, R., Marteau, P., Alric, M., Fonty, G., and Huis in 't Veld, J.H.J., A computer-controlled system to simulate conditions of the large intestine with peristaltic mixing, water absorption and absorption of fermentation products. Appl. Microb. Biotechn. 53: 108-114, 1999**), (**Venema, K., Van Nuenen, M., Smeets-Peeters, M., Minekus, M. and Havenaar, R., TNO's in vitro large intestinal model: an excellent screeening tool for functional food and pharmaceutical research, Ernährung/Nutrition 24 (12): 558-564, 2000**).** This model has been validated successfully with regards to the number and ratio of the various micro organisms which are similar in composition and metabolic activity with that of the human (or animal) colon. Furthermore, it has been validated for the production of metabolites, such as short chain fatty acids (including iso-form), gasses, ammonia, and phenol compounds and used for studies on bioconversion of glucosinolates by the human colon microbiota **(**Krul, C., Humblot, C., Philippe, C., Vermeulen, M., Van Nuenen, M., Havenaar, R., Rabot, S., Metabolism of sinigrin (2-propenyl glucosinolate) by the human colonic microflora in a dynamic in vitro large-intestinal model, Carcinogenesis 23 (6): 1009-1016, 2002**).**

### Aim of the Present Examples

The aim of the present examples was to investigate the influence of conjugated linoleic acid (CLA) and lactic acid bacteria (Lactobacillus acidophilus and Bifidobacterium bifidum), alone or in combination, on the microbiota activity in the large intestine, with respect to the production of SCFA, lactate, and protein-fermentative metabolites (e.g. BCFA, ammonia). In addition, the composition of the microbiota with respect to health-promoting microorganisms, (potentially) toxic and pathogenic microorganisms was investigated. Furthermore, the production of secondary bile acids and the effect of CLA and/or LAB on this was studied as well. The experiments were performed in the in vitro model of the large intestine (TIM-2), and studied in a microbiota that has been treated with antibiotics.

### Materials and Methods

### Test-products:

### Test-product #1

Name: conjugated linoleic acid (triglyceride of fatty acids, approximately 80 % by weight of the fatty acids are CLA, approximately 50 % by weight of the CLA is c9,t11-CLA, approximately 50 % by weight of the CLA is t10,c12-CLA) - Short name: CLA

### Test-product #2

Name: Lactobacillus acidophilus
from Variati & Co.

### Test-product #3 (Lot# 645297H)

Name: Bifidobacterium bifidum
from Variati & Co.

### Test system: TNO large-intestinal model (TIM-2) and origin of microbiota

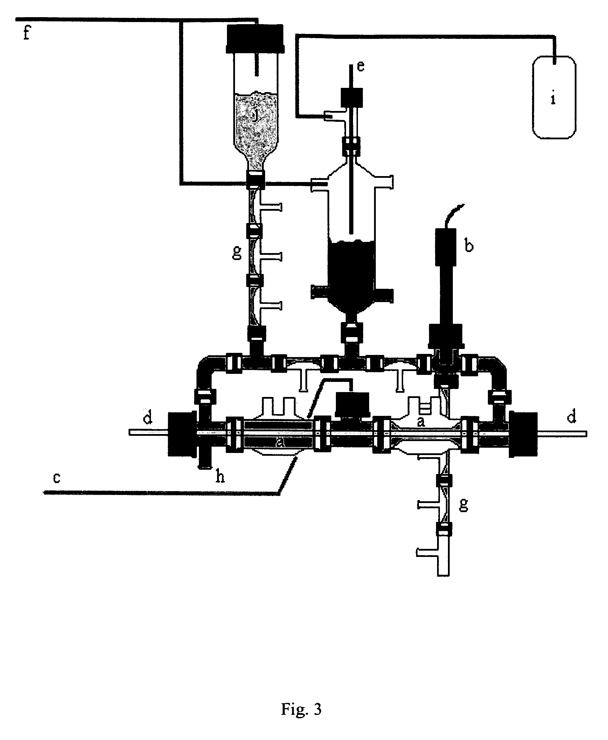

The experiments were performed in the dynamic in vitro system of the large intestine (TIM-2) as described above and presented in Fig. 2 and Fig. 3.

Fig. 3 is a schematic representation of TIM-2. The letters have the following meaning: a: peristaltic compartments; b: pH-electrode; c: alkali pump; d: dialysis liquid circuit with hollow fibers; e: level-sensor; f: N₂ gas inlet; g: in- or outlet valves; h: sampling-port; i: gas outlet; j: 'ileal delivery' container.

The model was inoculated with a standardized, active intestinal microbiota originating from healthy human adults (as described in: Venema, K., Van Nuenen, M., Smeets-Peeters, M., Minekus, M. and Havenaar, R. (2000). TNO's in vitro large intestinal model: an excellent screeening tool for functional food and pharmaceutical research. Emahrung/Nutrition 24 (12): 558-564). This assured that a microbiota identical in composition was used throughout the project. Prior to the addition of the test-products, the microbiota was treated with clindamycin at 15 µg/ml in a 16 h adaptation phase.

### Conduct of the TIM-2 experiments

In the study, which took from Monday to Friday (with a 16 h adaptation period, and 3 days of addition of the test-substrate), the composition of the colon microbiota was followed in time after intake of the test compounds during several days at an interval of 24 h. to study the effect on the compounds on the microbiota composition and activity. Differences in the speed of fermentation between the beginning and at the end of the long-term study might indicate the adaptation or selection of the microbiota to the test compounds.

### Sampling and analyses

Before and at the end of the fermentation experiments samples were taken from the lumen of the model, and from the dialysis liquid. These samples were analysed gas-chromatographically on the concentrations of SCFA and lactate. Furthermore, samples were taken for analyses on the protein-fermentative metabolites ammonia, and branched-chain fatty acids (BCFA). This indicated the balance between the production of health-promoting and toxic metabolites.

Additional samples were taken from the lumen of the colon model at the start and end of the fermentation experiments for analysis of the composition of the microbiota using non-selective and selective culture plates for the total anaerobic counts and the numbers of Bacteroides, Bifidobacterium, Lactobacillus, Enterococcus, (sulphite reducing) Clostridium, and Enterobacteriaceae. Analysis of the composition of the microbiota indicated the bacterial genera which are selectively stimulated or suppressed by the test compound(s).

Additionally, the volume of gas produced by the microbiota was measured. It should be noted that in TIM-2 strictly the gas produced by the microbiota is measured. The contribution of CO₂ by secretion of bicarbonate into the lumen of the large intestine by the intestinal epithelium is omitted in the model. Unforntunately, the small amount of gas produced (see Results) precluded analyses with respect to the composition of the gas.

Additional samples were taken at the start and at the end of the fermentation experiments for analyses with respect to the production of secondary bile acids (using FT-MS).

The objective of the present examples was to study the effect of the test-compounds on the activity and composition of the microbiota (after treatment with an antibiotic). The test-parameters were:
a) no test-product (control)
b) CLA at concentration 4.5 g/day
c) LAB at concentration 1*10⁸g cfu/day
d) CLA 4.5 g/day + LAB 1*10⁸ cfu/day

The dose of CLA was based on an anticipated dose per day. The dose of LAB is based on the combination of an anticipated dose per day plus the estimated survival of these strains during transit through the upper GI tract (stomach and small intestine). Assuming an intake of approx 1*10⁹ cfu (100 ml x 1*10⁷ cfu/ml) and a survival of approx. 10%, the estimated amount of viable cells reaching the colon is 1*10⁸ cfu. All experiments were performed in duplicate.

### Results and discussion

### Pilot experiments

In a short series of pilot experiments the behaviour of the test-compounds (CLA and LAB strains) and the optimal concentration of the antibiotic were established. Based on these results the final protocol was prepared.

### TIM-2 experiments

TIM-2 experiments were performed using a microbiota that was treated with clindamycin during the 16 hour stabilization period preceeding the addition of the test-compounds. The antibiotic was added to the lumen of the in vitro model, as well as the dialysate to sustain the concentration of the compound over the full adaptation period.

Duplicate experiments were carried out with the following test-parameters:
a) no test-product (control)
b) CLA at concentration 4.5 g/day
c) LAB at concentration 1*10⁸ cfu/day
d) CLA 4.5 g/day + LAB 1*10⁸ cfu/day

Samples from the lumen and dialysate were analyzed for production of microbial metabolites, composition of the microbiota and conversion of primary bile acids into secundary bile acids.

### Production of microbial metabolites

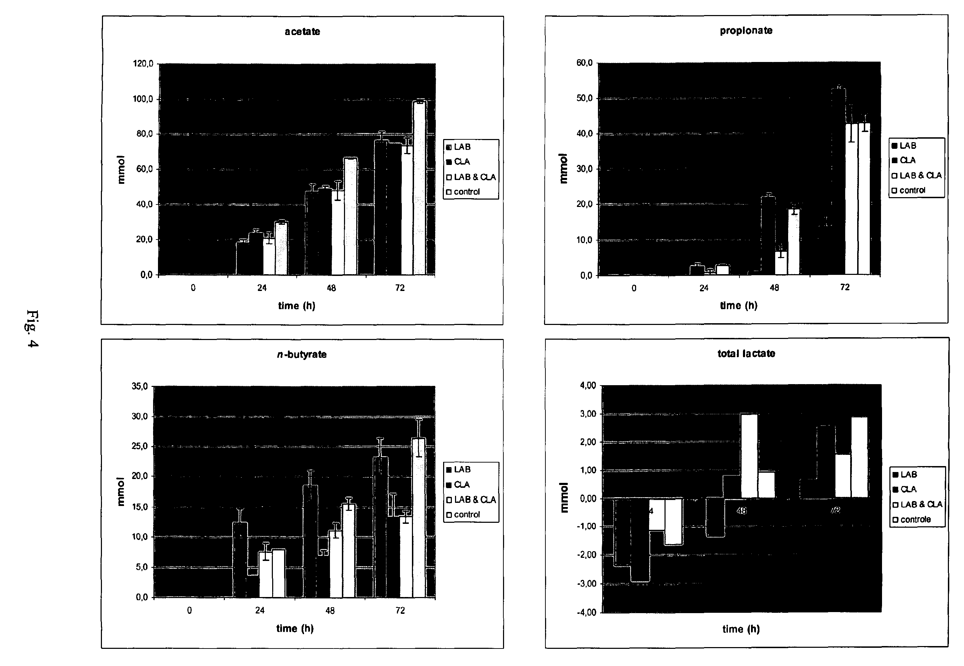

Fig. 4 shows the cumulative production of short-chain fatty acids (SCFA, acetate, propionate and butyrate) and lactate during the different experiments. These results show that there is a reduction in production of acetate upon addition of LAB, CLA or the combination of the two. Propionate production is slightly induced by CLA, and reduced by LAB. When these are added in combination, there is no influence on production when compared to the control. Butyrate is reduced in those experiments where CLA was added. Lactate production is more variable. This is mainly because there is only limited lactate production (in fact, immediately after the adaptation phase there is consumption of lactate, evidenced by its 'negative production'). Cumulative lactate production maximally reaches 3 mmol, versus 100 mmol, 60 mmol and 25 mmol for acetate, propionate and butyrate respectively.

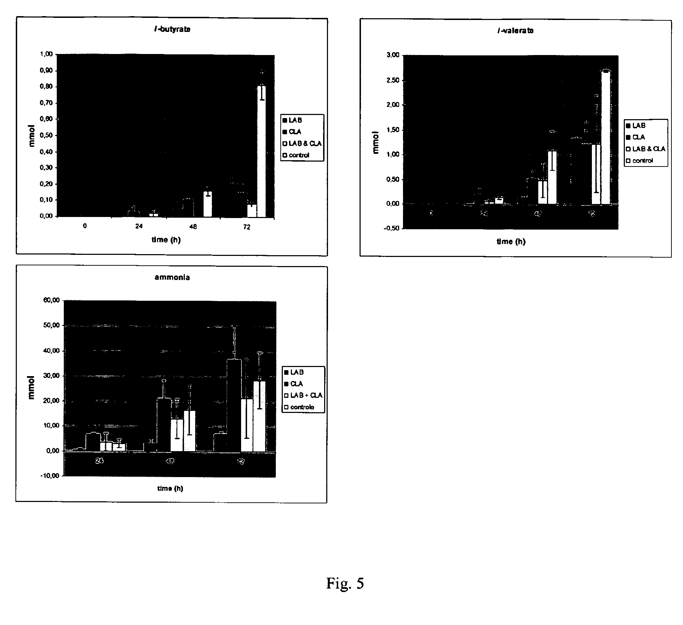

Fig. 5 shows the cumulative production of metabolites produced from protein fermentation, a process which is generally believed to be putrefactive and lead to production of toxic metabolites (amongst others ammonia). From this figure it can be observed that generally protein fermentation was greatly reduced by the addition of LAB (reduction of ammonia and the branched-chain fatty acids (BCFA) *i*-butyrate and *i*-valerate, of which the latter two are biomarkers for protein fermentation as they are derived from branched-chain amino acids). CLA (either alone or in combination with LAB) has a similar effect on reduction of BCFA production, but no (CLA) or a less clear (CLA+LAB) effects on production of ammonia. It is our experience that ammonia production is more difficult to influence than production of other putrefactive metabolites, basically because of the great amount of transaminase reactions carried out by the microbiota. In general however, it can be concluded that the CLA and LAB (alone and in combination) reduce protein fermentation and reduce production fo toxic metabolites.

Gas volume was measured as well, but no differences between the experiments were observed (data not shown). The little amount of gas produced precluded chemical analysis.

### Microbiota composition

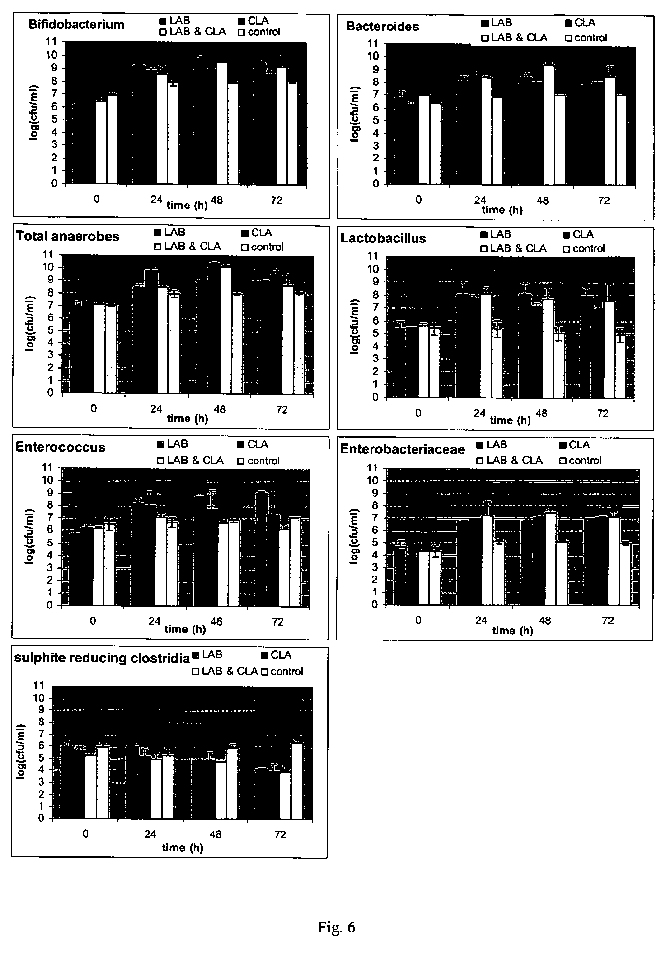

Fig. 6 displays the composition of the microbiota in time. At the start of the experiments, relative low numbers of microorganisms are present, due to the treatment with clindamycin. In the control experiment, most microbial groups slowly increase in time, due to recovery after the antibiotic treatment. In the experiments with LAB, and CLA+LAB, recovery of the *Bifidobacterium* group and the *Lactobacillus* group is more pronounced than in the control, as expected because these microorganisms are added to the system. Although not as pronounced as with those treatments where LAB was added, CLA addition alone also seems to favour recovery of these microbial groups in time. Also recovery of Bacteroides, total anearobes, Enterobacteriaceae and Enterococcus progresse faster when LAB, CLA or both are added (except for LAB+CLA addition with Enterococcus). Slufite reducing clostridia (involved in the conversion of sulfur-containing compounds (sulfate, sulfur-containing amino acids) into the toxic hydrogen sulfide are reduced pronouncedly upon addition of the test-compounds (alone or in combination). Taken together, the effects observed in our *in vitro* model point into the direction of a more beneficial composition of the microbiota after addition of the test-compounds following antibiotic administration.

### Conversion of bile acids

The influence of CLA and LAB on the conversion of primary bile acids into secundary bile acids was studied as well. For this, samples were taken from the lumen of TIM-2 and analyzed using FT-MS.

The bile acids that were measured using this method were: Cholic acid (CA), Deoxycholic acid (DC), Chenodeoxycholic acid (CDC), Lithocholic acid (LCA) and Ursodeoxycholic acid (UDCA). Trace amounts of the conjugated bile salt (tauro- or glycoconjugates) were observed as well.

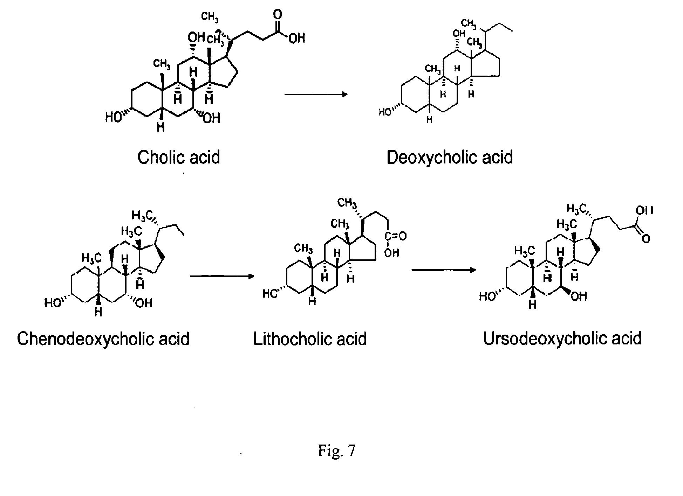

Conversion of the primary bile acids CA -> DC, and CDC -> LCA -> UDCA is shown in Fig. 7.

The concentration of measured bile acids in the different experiments is displayed in Table 1. From the table it can be observed that CLA (alone or in combination with LAB) addition resulted in high amounts of the primary bile acids CA and CDC. The highest amounts were observed when CLA was given together with the LAB. Also, the combination of CLA+LAB resulted in lowest amounts of the secundary bile acid DC. CLA or LAB alone did not seem to have an effect on this. Similarly, the combination of CLA and LAB reduced LCA amounts, whereas the individual addition of CLA or LAB only lowered the amount of this secundary bile acid marginally when compared to control. Lastly, CLA reduced the amount of the secundary bile acid UDCA by about 40%. Strikingly, addition of LAB increase amounts of this bile acid. Addition of both CLA and LAB seemed to results in normalization by CLA of the increase caused by LAB, to a level similar to that produced in the control experiment.

Taken together, the results indicate that CLA (either alone, but synergistically for some bile acids with LAB) seemed to beneficially influence the amounts ofprimary versus secundary bile acids in the *in vitro* model. Since the secundary bile acids are believed to be involved in amongst others stimulation in the onset of colon cancer, this is considered to be beneficial for gut health.

**Table 1. Concentration of the primary (green) and secundary (red) bile acids in the lumen samples of TIM-2 taken at 72 hours.**

| | CA | DC | CDC | LCA | UDCA |
|---|---|---|---|---|---|
| Control | 162 | 30800 | 5 | 5668 | 616 |
| CLA | 2035 | 35500 | 1200 | 4090 | 383 |
| LAB | 293 | 35000 | < 4 | 4635 | 1120 |
| CLA+LAB | 7346 | 14400 | 2060 | 1575 | 567 |

### Conclusions and recommendations

The aim of the present examples was to investigate the influence of conjugated linoleic acid (CLA) and lactic acid bacteria (*Lactobacillus acidophilus* and *Bifidobacterium bifidum*), alone or in combination, on the microbiota activity in the large intestine, with respect to the production of SCFA, lactate, and protein-fermentative metabolites (*e.g.* BCFA, ammonia). In addition, the composition of the microbiota with respect to health-promoting microorganisms, (potentially) toxic and pathogenic microorganisms was investigated. Furthermore, the production of secondary bile acids and the effect of CLA and/or LAB on this was studied as well.

Conclusions that can be drawn from the results are:
- LAB reduced the production of the protein fermentative metabolites BCFA and ammonia;
- CLA reduced the production of the protein fermentative metabolites BCFA only. Ammonia was unaffected;
- When LAB were added, an increase in *Lactobacillus* and *Bifidobacterium* counts were observed, as expected, but in addition the composition of the microbiota after antibiotic treatment was normalized quicker and better when compared to control;
- CLA also normalized the microbiota after antibiotic treatment but to a lesser extent with respect to the counts of *Lactobacillus* and *Bifidobacterium,* most likely due to the fact that in this case these microorganisms were not added;
- The production of secundary bile acids was reduced in the presence of CLA, and at the same time, the amount of primary bile acids was higher than that in the control experiment. The effect was highest when CLA was added together with LAB.

## Claims

1. Use of CLA to support the recovery of a defective intestinal flora of a mammal.

2. Use of CLA to keep up or support a balanced intestinal flora in a mammal.

3. The use according to any of claims 1 or 2 wherein the mammal is a human.

4. The use according to any of claims 1 to 3 wherein 1 to 10 g of CLA (calculated as free acid) are administered per individual per day.

5. The use according to any of claims 1 to 4 wherein the intestinal flora has been or is influenced negatively by at least one antibiotic.

6. A composition comprising CLA for use as a medicament for treating or for preventing a defective intestinal flora of a mammal.

7. The composition according to claim 6 wherein the mammal is a human.

8. The composition according to any of claims 6 or 7 wherein the use comprises administering 1 to 10 g of CLA (calculated as free acid) per individual per day.

9. The composition according to any of claims 6 to 8 wherein the intestinal flora has been or is influenced negatively by at least one antibiotic.
